# EUROPÄISCHE PATENTANMELDUNG

(11) **EP 1 525 898 A1**
(43) Veröffentlichungstag der Anmeldung: **27.04.2005**
(21) Anmeldenummer: 03024341.4
(22) Anmeldetag: 23.10.2003
(51) Int. Cl.: A61M 29/02

(54) **Verfahren und Anordnung für einen Applikationskatheter**

(71) Anmelder: Acrostak Corp., 8409 Winterthur (CH)
(72) Erfinder: Schwager, Michael, 8404 Winterthur (CH); Haase, Karl Konstantin, Prof. Dr. med., 67346 Speyer (DE); Herdeg, Christian, Priv. Doz. Dr. med., 72072 Tübingen (DE)
(74) Vertreter: Weiss, Wolfgang

(57) **Zusammenfassung**

Die Erfindung betrifft ein Verfahren zur Verabreichung eines Medikamentes in ein Blutgefäss (1) mittels eines Ballonkatheters (2) mit mindestens einem Flanken (3) aufweisenden Ballon (4), wobei die Einströmrichtung des Medikamentes in einem Winkel α von 0° bis 89° erfolgt und der Winkel a aus der Strömungsrichtung des Blutes im Blutgefäss (1) und der Flanke (3) des Ballons (4) gebildet wird.

## Beschreibung

Die Erfindung betrifft ein Verfahren und eine Anordnung für einen Applikationskatheter zur Verabreichung eines Medikamentes in ein Blutgefäss mittels eines Ballonkatheters mit mindestens einem Flanken aufweisenden Ballon.

Derartige Verfahren und Anordnungen von Applikationskathetern dienen dazu, ein Medikament an einem gewünschten Ort zu verabreichen mit dem Ziel, eine optimale Wirkung zu erreichen. Dieser Idee der lokalen Applikation liegt der Gedanke zugrunde mit einer hohen lokalen Wirkstoffkonzentration und geringeren systemischen Nebenwirkungen bessere Ergebnisse zu erzielen.

Zum Stand der Technik gehören die Anwendung von mit einem Medikament beschichtete Stents. Weiterhin sind Ballonsysteme bekannt.

Aus der Veröffentlichung US 2003/0045860 A1 ist ein derartiges Verfahren und eine Anordnung dieser Gattung bekannt geworden. Dabei handelt es sich um zwei sequentiell angeordnete ausdehnbare Ballone, deren Aussenwände sich an die Innenwand des zu behandelnden Gefässes andrücken. Die zu behandelnde Stelle des Blutgefässes befindet sich zwischen den beiden Ballons. Der in dem Zwischenbereich der beiden Ballons angeordnete Bereich mit wesentlich kleinerem Durchmesser ist perforiert. Durch diese Perforationen wird das Medikament in an sich bekannter Weise eingegeben, dabei verläuft die Strömungsrichtung des Medikamentes direkt, d.h. in einem Winkel α von 90°, zur Strömungsrichtung des Blutes. Das Medikament gelangt somit mit maximalem Druck auf die zu behandelnde Stelle des Blutgefässes.

Ein anderes Verfahren bzw. eine andere Anordnung eines Applikationskatheters ist in der Veröffentlichung US 5,611,775 beschrieben. Hierbei handelt es sich um eine aus einem ausdehnbaren Ballon bestehenden Vorrichtung, wobei der Ballon an der Kontaktfläche des Ballons mit der Innenwand des Gefässes zur Eingabe des Medikamentes perforiert ist.

Alle diese bekannten Applikationskatheter sind mit Nachteilen verbunden. Diese Verfahren weisen nämlich Grenzen im Hinblick auf die Effizienz der kathetergestützten lokalen Therapie durch einen generell niedrigen Wirkstofftransfer und ein schnelles Auswaschen der Substanz aus der Gefässwand auf. Da die physikalischen Möglichkeiten, einen Wirkstoff mittels Katheter in die Gefässwand einzubringen, limitiert sind und sich zwischen den beiden Möglichkeit der passiven Applikation (Gefässwand wird im Wirkstoff gebadet) einerseits und der aktiven Applikation (Wirkstoff wird mit maximal möglichem Druck in die Gefässwand eingebracht) bewegen, wurde nach einem geeigneten Verfahren und einer geeigneten Anordnung gesucht, die es erlauben, sich kontinuierlich zwischen den beiden aktiven und passiven Möglichkeiten zu bewegen. Im übrigen ist bei diesen bekannten Katheteranordnungen eine vollständige Entleerung des Behandlungsraumes nicht gewährleistet mit der Folge, dass das Medikament teilweise vom Blut weggespült wird und vom Körper abgebaut werden muss. Es entstehen dadurch nicht erwünschte systemische Verbreiterungen des Medikamentes im Körper.

Die mit der vorliegenden Erfindung verbundene Aufgabe liegt somit darin, ein Verfahren und eine Anordnung für einen Applikationskatheter zur Verabreichung eines Medikamentes in ein Blutgefäss mittels eines Ballonkatheters vorzuschlagen, bei dem längere Applikationszeiten unter Vermeidung eines Gefässtraumas möglich sind. Weiterhin soll eine vollständige Entleerung des Applikationsraumes erreicht werden können. Insbesondere soll eine Lösung vorgeschlagen werden, die entsprechend des pharmakologischen Profils einer medikamentösen Substanz spezifisch für eine ballongestützte Anwendung geeignet ist.

Diese Aufgabe wird erfindungsgemäss dadurch gelöst, dass die Einströmrichtung des Medikamentes in einem Winkel α von 0° bis 89° erfolgt, wobei der Winkel aus der Strömungsrichtung des Blutes im Blutgefäss und der Flanke mindestens eines der Ballons gebildet wird. Ferner wird die Aufgabe dadurch gelöst, dass der Ballon ein zusätzliches Lumen aufweist, dessen offenes Ende zwischen dem distalen Ende des Ballons und dem proximalen Ende des benachbarten Ballons angeordnet ist.

Mittels dem erfindungsgemässen Verfahren wird ermöglicht, einen Wirkstoff in hoher Konzentration in homogener und nicht gefässtraumatisierender Weise über einen definierten Zeitraum vor Ort abzugeben. Beispielsweise ist für die Abgabe von Paclitaxel diese Tatsache entscheidend. Pactlitaxel hinterlässt seine zelluläre Wirkung, nämlich eine Änderung des Zytoskeletts, auch dann noch, wenn die Substanz selbst schon längst ausgewaschen ist. Bei dem erfindungsgemässen Verfahren werden die schädlichen Nebenwirkungen vermieden, zumindest reduziert. In diesem Zusammenhang werden beispielhaft folgende weitere für dieses erfindungsgemässe Verfahren geeignete Substanzen genannt: Sirolimus, Everolimus und Stammzellen.

Mit dem Medikament wird auch gleichzeitig der distale und proximale Ballon in folgender Weise entfaltet und aufgeblasen:

Das Medikament wird durch einen Schaft in den Katheter eingespritzt. Dadurch entfaltet sich zuerst der Ballon in seine Form und entwickelt durch seine hantelförmige Ausgestaltung (distaler und proximaler Ballon) eine dichtende Wirkung auf beide Seiten des zu behandelnden Bereiches. Das der Ballon sich zuerst entfaltet, erreicht man technisch nur dadurch , dass der Gesamtquerschnitt der Perforationen genügend klein ist. Es soll damit vermieden werden, dass das Medikament ohne Entfaltung des Ballons aus den Perforationen heraustritt. Das Entleeren des Applikationsraumes geschieht durch Anwendung von Unterdruck. Das Medikament wird durch die Perforationen wieder zurückgezogen. Dabei muss das Volumen des Applikationsraumes genügend klein gegenüber dem Gesamtvolumen des Katheters gewählt werden.

In vorteilhafter Weise sind die zu den behandelten kranken Gefässbereichen gerichteten Ballonflanken mit Perforationen versehen, wobei die Steilheit der Flanken einstellbbar sind.

Die Erfindung zeichnet sich dadurch aus, dass der Winkel α je nach Anwendung und/oder Medikamentenart einstellbar ist. Somit bestimmt die Steilheit der perforierten Flanke eines Ballons den Druck, mit dem das Medikament auf den zu behandelnden Bereich aufgebracht wird. Damit wird es somit möglich, die Eindringtiefe und die Wirkungsdauer eines Medikamentes einzustellen. Mit dieser Möglichkeit den Winkel einzustellen kann die Katheteranordnung spezifisch auf die zu verabreichende Substanz und die spezielle Anwendung ausgerichtet werden.

Gemäss einer bevorzugten Ausführung weist die Katheteranordnung ein zusätzliches Lumen auf, dessen offenes Ende zwischen dem distalen Ende eines Ballons und dem proximalen Ende des benachbarten Ballons angeordnet ist. Damit wird eine Beeinflussung des Blutflusses im Blutgefäss nahezu vermieden. Durch geeignete Mittel wird die Kontinuität der Blutströmung gewährleistet..

Die Erfindung wird im folgenden mittels eines Ausführungsbeispiels beschrieben. Es zeigen:
- Fig.1:: eine schematische Schnittdarstellung einer Katheteranordnung,
- Fig.2:: die Einzelheit B von Figur 1 vergrössert dargestellt
- Fig.3:: ein Querschnit entlang des Schnittes C - C von Figur 1
- Fig.4:: eine schematische Schnittdarstellung einer Katheteranordnung mit einem zusätzlichen Lumen und
- Fig.5:: ein Querschnitt entlang des Schnittes A - A von Figur 4

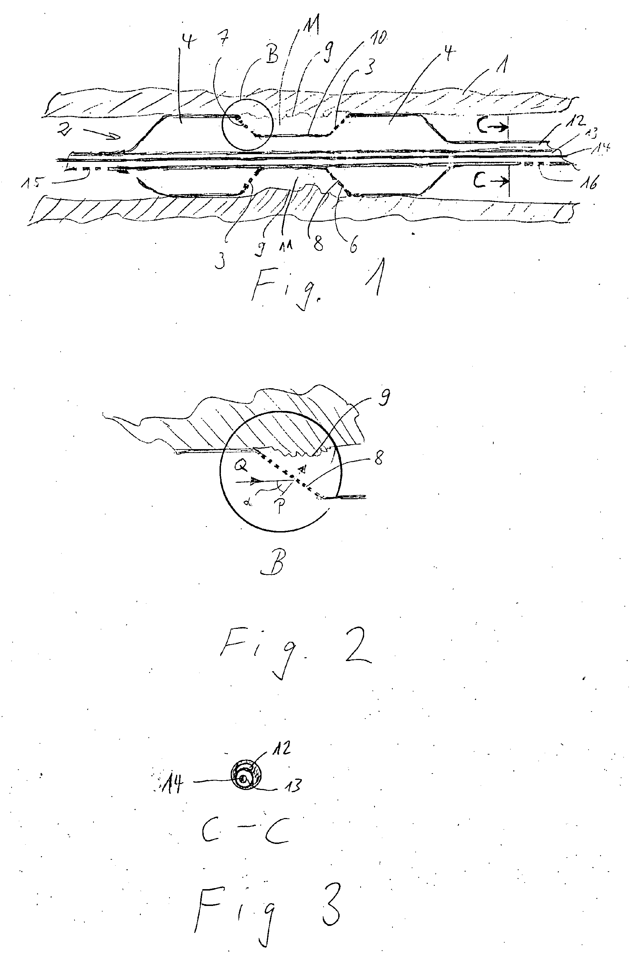

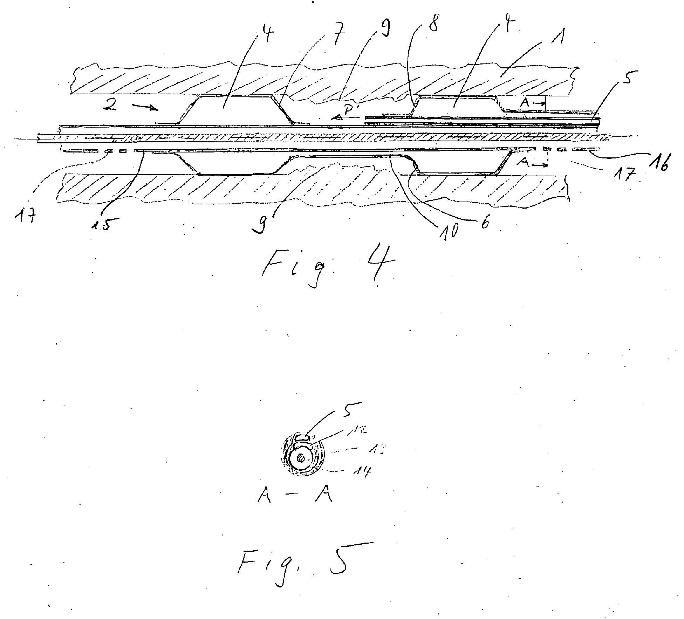

Das Bezugszeichen 1 betrifft das Blutgefäss 1, welches einen zu behandelnden Bereich 9 aufweist. In dem Blutgefäss 1 ist ein aus zwei Ballons 4 bestehender Ballonkatheter 2 in der Weise plaziert, dass sich der zu behandelnde Bereich 9 zwischen den beiden Ballons 4 befindet. Der Querschnitt der Ballons 4 ist grösser als der Querschnitt 10 des Bereiches 11 zwischen den Ballons 4. Die Verbindung zwischen den Ballons 4 und dem Zwischenbereich 11 erfolgt mittels Flanken 3, sowohl am proximalen 7 als auch am distalen 6 Ende der Ballons 4 Flanken 3. Die. der zu behandelden Bereiche 9 zugewandten Wände der Flanken 3 sind erfindungsgemäss zur Zugabe von Medikamenten perforiert und bewusst nicht die Wand des Zwischenbereichs 11. In Figur 2 ist die Einzelheit B von Figur 1 dargestellt. Der Winkel α ist definiert als Winkel zwischen der Richtung Q der Blutströmung im Gefäss 1 und der Richtung P der Medikamentenströmung. Das Medikament wird durch die Perforationen 8 in den Flanken 3 eingegeben. Aufgrund des bei der Herstellung des Ballonkatheters einstellbaren Winkels α kann der Auftreffdruck und damit die Wirkungsintensität (Wirkungsdauer und Wirkungstiefe) je nach Anwendung und Medikament spezifisch eingestellt werden. In der Figur 3 ist ein Querschnitt des Schnittes C - C von Figur 1 gezeigt. Zu sehen ist das Inflationslumen 12, das Drahtlumen 13 und der Führungsdraht 14.

In Figur 4 weist die Katheteranordnung ein zusätzliches Lumen 5 zur separaten Medikamentenzugabe auf. Durch das zusätzliche Lumen 5 wird das Medikament in den Zwischenraum 11 parallel zur Blutströmungsrichtung P in Richtung P' eingebracht. Denkbar ist auch ein gekrümmtes in den Behandlungsraum weisendes Ende des zusätzlicher Lumens 5. Das Medikament wird in einem gewünschten Winkel und einen damit verbundenen Auftreffdruck auf die zu behandelnde Stelle zugegeben. Die Schnittzeichnung in Figur 5 gemäss des Schnittes A - A von Figur 4 zeigt das zusätzliche Lumen 5, das Inflationslumen 12, das Drahtlumen 13 und das Lumen für den Führungsdraht 14. Sowohl am distalen 6 als auch am proximalen 7 Ende der Katheteranordnung sind Perforationen 17 angebracht. Diese Perforationen 17 dienen zur Aufrechterhaltung und damit zum Ausgleich der Blutströmung im Blutgefäss 1.

Das erfindungsgemässe Verfahren kann auch zur Behandlung von anderen Organsystemen, wie beispielsweise Trachea, Bronchien, Speiseröhre, ableitende Harnwege oder Gallenkanäle mittels spezifischer Medikamente verwendet werden.

Die mit der Erfindung bestehenden Vorteile liegen insbesondere darin, dass ein Verfahren und eine Katheteranordnung für eine spezifische Behandlung bezogen auf die Anwendung und die Medikamenteigenschaften vorgeschlagen werden.

### Bezugszeichen

- 1: Blutgefäss
- 2: Ballonkatheter
- 3: Flanke
- 4: Ballon
- 5: Lumen
- 6: distales Ende
- 7: proximales Ende
- 8: Perforationen
- 9: Behandelnder Bereich
- 10: Querschnitt vom Zwischenraum 11
- 11: Zwischenraum
- 12: Infaltionslumen
- 13: Drahtlumen
- 14: Draht
- 15: proximales Ende
- 16: distales Ende
- 17: Perforationen

- P: Einströmrichtung
- Q: Fliessrichtung des Blutes im Gefäss

- α: Winkel

## Patentansprüche

1. Verfahren zur Verabreichung eines Medikamentes in ein Blutgefäss (1) mittels eines Ballonkatheters (2) mit mindestens einem Flanken (3) aufweisenden Ballon (4), **dadurch gekennzeichnet, dass** die Einströmrichtung P des Medikamentes in einem Winkel α von 0° bis 89° erfolgt, wobei der Winkel α aus der Strömungsrichtung Q des Blutes im Blutgefäss (1) und der Flanke (3) des Ballons (4) gebildet wird.

2. Verfahren zur Verabreichung eines Medikamentes nach Anspruch 2, **dadurch gekennzeichnet, dass** der Winkel α je nach Anwendung und/oder Medikamentenart einstellbar ist.

3. Verfahren nach Anspruch 2 oder 3, **dadurch gekennzeichnet, dass** die Ausbildung der Katheteranordnung spezifisch auf die zu verabreichende Substanz ausgebildet wird.

4. Verfahren nach mindestens einem der Ansprüche 1, 2 oder 3, **dadurch gekennzeichnet, dass** der Gesamtquerschnitt der Perforationen 8 derart ausgebildet sind, dass die Ballone 4 hantelförmig entfaltbar und aufblasbar sind.

5. Katheteranordnung nach dem Verfahren von mindestens einem der Verfahrensansprüche 1 bis 3, **dadurch gekennzeichnet, dass** der Ballon (4) ein zusätzliches Lumen (5) aufweist, dessen offenes Ende zwischen dem distalen Ende (6) eines Ballons (4) und dem proximalen Ende (7) des benachbarten Ballons (4) angeordnet ist und in den Behandlungsraum weist.

6. Katheteranordnung nach Anspruch 4, **dadurch gekennzeichnet, dass** sowohl am proximalen (15) als auch am distalen (16) Ende der Katheteranordnung Perforationen (17) angeordnet sind.

7. Katheteranordnung nach Anspruch 4, **dadurch gekennzeichnet, dass** mindestens eine Flanke (3) eines Ballons (4) Perforationen (8) zur Verabreichung eines Medikamentes aufweist.

8. Katheteranordnung nach Anspruch 4 oder 5, **dadurch gekennzeichnet, dass** die Flanke (3) mit der Fliessrichtung Q des Blutes einen Winkel α bildet, wobei der Winkel 0° bis 89° beträgt.
